# EUROPEAN PATENT SPECIFICATION

(11) **EP 3 360 970 B1**
(45) Date of publication and mention of the grant of the patent: **30.10.2019**
(21) Application number: 17000209.1
(22) Date of filing: 10.02.2017
(51) Int. Cl.: C12Q 1/6851, G01N 21/64

(54) **A METHOD FOR REDUCING QUANTIFICATION ERRORS CAUSED BY AN OPTICAL ARTEFACT IN DIGITAL POLYMERASE CHAIN REACTION**
VERFAHREN ZUR REDUZIERUNG VON QUANTIFIZIERUNGSFEHLERN, DIE DURCH EIN OPTISCHES ARTEFAKT IN EINER DIGITALEN POLYMERASEKETTENREAKTION VERURSACHT SIND
PROCÉDÉ DE RÉDUCTION D'ERREURS DE QUANTIFICATION PROVOQUÉES PAR UN ARTÉFACT OPTIQUE DANS UNE RÉACTION EN CHAINE DE POLYMÉRASE NUMÉRIQUE

(43) Date of publication of application: 15.08.2018
(73) Proprietor: Roche Diagnostics GmbH, 68305 Mannheim (DE); F. Hoffmann-La Roche AG, 4070 Basel (CH)
(72) Inventor: BETSCHART, Florian, 6440 Brunnen (CH); WASER, Patrick, 6004 Luzern (CH)
(74) Representative: Teschemacher, Andrea

(56) References cited:
- EP-A1- 3 299 470
- WO-A1-2014/153369
- WO-A2-2016/127124
- US-A- 6 035 072
- US-A1- 2013 302 792
- ANDREW C. HATCH ET AL: "1-Million droplet array with wide-field fluorescence imaging for digital PCR", LAB ON A CHIP, vol. 11, no. 22, 1 January 2011 (2011-01-01), page 3838, XP55063496, ISSN: 1473-0197, DOI: 10.1039/c1lc20561g

## Description

The present invention relates to a method for reducing quantification errors caused by an optical artefact in digital polymerase chain reaction (dPCR) and to a method for determining the amount or concentration of a nucleic acid of interest in a sample with dPCR.

For many biological, biochemical, diagnostic or therapeutic purposes, it is necessary to accurately and precisely determine the amount or concentration of a nucleic acid in a sample. dPCR is a rather new approach to nucleic acid detection and quantification that offers an alternative method to conventional real-time quantitative PCR for absolute quantification of nucleic acids and rare allele detection. dPCR works by partitioning a sample of nucleic acids into many individual, parallel PCR reactions; some of these reactions contain the target molecule (positive) while others do not (negative). Following PCR analysis, the fraction of negative reactions is used to generate an absolute count of the number of target molecules in the sample. One of the key advantages of dPCR over real-time PCR is its superior accuracy of quantification. This advantage relies on inherent properties of dPCR as quantification only requires correct counting of positive partitions and the knowledge of the theoretical partition volume (the count number is not very sensitive to PCR efficiency). A quantification standard is not required. This eliminates potential quantification errors caused by the standard itself.

The prior art provides methods in order to identify incorrect positive or negative counts and for calibrating or normalizing signals in droplet-based assay (US 2013/0302792 A1). This normalization should improve the separation between positive and negative counts. Hence the normalization reduces the risk of false positive or negative counts. The ultimate goal is to improve the accuracy and precision of the determination of the nucleic acid concentration by correcting the signal obtained for the nucleic acid.

WO2016127124 discloses methods for calibration of instruments used for biological optical imaging, including taking control measurements to account for errors.

However, the methods of the prior art do not account for quantification errors in PCR due to situations, in which the optical determination is impaired by an artefact.

Accordingly, there is a need for methods of quantifying a nucleic acid of interest by dPCR, which reduce quantification errors caused by an optical artefact. The object of the present invention was to provide those methods.

The problem was solved by methods based on digital polymerase chain reaction (dPCR) in which the distribution of the optical signals in each reaction area is analyzed and a reaction area with an optical artefact is excluded from the calculation of the amount or concentration of the nucleic acid of interest.

Accordingly, the present invention provides a highly accurate and precise method to quantify a nucleic acid by dPCR, which allows for more precise and accurate determination of the amount or concentration of a nucleic acid of interest in a sample. Particularly, the above methods allow for exclusion of reaction areas with optical artefacts e.g. due to impurities in the reaction area (e.g. dirt) or technical failures in the optional measurements.

In a first aspect, the present invention relates to a method for reducing quantification errors caused by an optical artefact in digital polymerase chain reaction (dPCR), wherein the amount or concentration of a nucleic acid of interest is quantified in an array of reaction areas, the method comprising
a) providing an array of reaction areas used in dPCR;
b) determining the distribution of the optical signals in each reaction area;
c) identifying a reaction area as invalid, if the optical signals in the reaction area determined in step b) are unequally distributed in the reaction area; and
d) eliminating the reaction area identified as invalid from calculating the amount or concentration of the nucleic acid of interest.

As detailed above, a method to reliably determine the amount or concentration of a nucleic acid is of particular relevance in several industrial applications, e.g. in the medical field. For several aspects it may not only be necessary to clarify whether or not a nucleic acid is present in the sample, but it may be required to determine - as precisely and accurately as possible - the amount or concentration of the nucleic acid in the sample, e.g. a sample obtained from a patient or product. This might be of interest e.g. in the diagnosis of the severity of a disease or in environmental technology or quality control of products, e.g. in order to define contaminations or impurities.

Present dPCR methods focus on the determination of the number of nucleic acids present in the intended volume and do not account for optical artefacts.

dPCR (digital polymerase chain reaction, digital PCR or DigitalPCR) is a biotechnology refinement of conventional polymerase chain reaction methods that can be used to directly quantify and optionally clonally amplify nucleic acids including DNA, cDNA, RNA or mixtures thereof. The key difference between dPCR and traditional PCR (e.g. qPCR) lies in the method of measuring nucleic acids amount, with the former being a more precise and accurate method than PCR, though also more prone to error in the hands of inexperienced users. The smaller dynamic range of dPCR may require dilutions of the sample. dPCR also carries out a single reaction within a sample, however the sample is separated into a large number of partitions or reaction areas and the reaction is carried out in each partition or reaction area individually. This separation allows a more reliable collection and sensitive measurement of nucleic acid amounts. Moreover, the method allows for accurate quantification.

As detailed above, the dPCR sample is partitioned so that individual nucleic acid molecules within the sample are localized and concentrated within many separate regions (reaction areas). The partitioning of the sample allows to estimate the number of nucleic acids by assuming that the molecule population follows the Poisson distribution. As a result, each part will contain a negative or positive reaction ("0" or "1", respectively). After PCR amplification, nucleic acids may be quantified by counting the regions that contain PCR end-product positive reactions. In conventional quantitative PCR, the quantitation result may depend on the amplification efficiency of the PCR process. dPCR, however, is not dependent on the number of amplification cycles to determine the initial sample amount, eliminating the reliance on uncertain exponential data to quantify target nucleic acids and therefore provides absolute quantification.

The first aspect of the present invention relates to a method for reducing quantification errors caused by an optical artefact.

An optical artefact is an optical signal that arises from a source other than the intended one. In the context of the present invention it does not arise from the dPCR assay, but is any undesired or unintended alteration in the optical signal determined in an optical process. The artefact may either arise from a disturbing matter such as an impurity affecting the dPCR signal or from the technique or device used in the determination of the optical signal.

An artefact may be any kind of impurity including but is not limited to dirt, dust, a scratch, fluid splash e.g. separation silicon oil, hair, fiber, dirt from fingerprints, incorrect filling of a reaction area or defects in the partitions structure of the reaction area e.g. a partition that has only half depth due to certain reasons. The artefacts can be placed either on the lower or upper surface of the reaction area or inside the same.

Dirt refers to matter, which renders a product unclear or impure. Dirt is material (often small-sized particles or traces), which is unwanted in the eye of the user or viewer. Common types of dirt include without limitation dust (a general powder of organic or mineral matter), residues of e.g. soil, oil, grease etc., filth (foul matter such as excrement), grime (a black, ingrained dust such as soot) and soil (the mix of clay, sand, and humus which lies on top of bedrock).

The optical artefact may also be caused by modifications on the surface of the array, such as removal or addition of material on the surface (inside or outside) including without limitation a scratch, fluid splash e.g. separation silicon oil, hair, fiber, dirt from fingerprints, or a defect in the array structure e.g. a partition that has only half depth due to certain reasons. Finally, incorrect filling of a reaction area (e.g. only the reaction area is filing partially, only) may also cause the artefact.

Additionally or alternatively, the artefact may be due to technical problems during imaging. These artefacts are due to an unintended difference between the imaged source and the image. Reasons for the differences may be for example blooming, chromatic aberration, jaggies, aliasing, JPEG compression, moire or noise.

Blooming is an overflow of electrical charge that can spill onto existing pixels, causing overexposure in areas of an image.

Chromatic aberration is an effect resulting from dispersion in which there is a failure of a lens to focus all colors to the same convergence point. It occurs because lenses have different refractive indices for different wavelengths of light. The refractive index of transparent materials decreases with increasing wavelength in degrees unique to each. Chromatic aberration manifests itself as "fringes" of color along boundaries that separate dark and bright parts of the image, because each color in the optical spectrum cannot be focused at a single common point. Since the focal length *f* of a lens is dependent on the refractive index *n,* different wavelengths of light will be focused on different positions.

Jaggies or aliasing refers to the visible jagged edges on diagonal lines in a digital image. Pixels are square and because a diagonal line consists of a set of square pixels it can look like a series of stair steps when the pixels are large. Sharpening in post production will increase the visibility of jaggies.

JPEG compression is used for saving images. JPEG is the most common photo file format used to save digital photo files. However, JPEG gives a trade-off between image quality and image size. When saving a file as a JPEG, the image is compressed and quality is lost.

Moire is caused when an image contains repetitive areas of high frequency. These details can exceed the resolution of the camera. It looks like wavy colored lines on the image.

Noise shows up on images as unwanted or stray color specks, and noise is most commonly caused by raising the ISO of a camera. It will be most apparent in the shadows and blacks of an image, often as small dots of red, green, and blue. Noise can be reduced by using a lower ISO.

In the first step of the method of the first aspect, an array of reaction areas used in dPCR is provided.

The reaction area used in dPCR can be any array of reaction areas suitable for use in dPCR and includes without limitation a miniaturized chamber of a microarray or a nanoarray, a chamber of a microfluidic device, a microwell or a nanowell. The reaction area may be on a chip, in a capillary, on a nucleic acid binding surface or on a bead. Preferably the array is a microarray or on a chip.

There is a multitude of dPCR systems available, which may be used in the present invention. Commercialized digital PCR platforms include micro-well chip-based BioMark® dPCR from Fluidigm, through hole-based QuantStudio12k flex dPCR and 3D dPCR from Life Technologies, and droplet-based ddPCR (ddPCR) QX100 and QX200 from Bio-Rad® and RainDrop from RainDance®. The microfluidic-chip-based dPCR can have up to several hundred reaction areas per panel. Droplet-based dPCR usually has approximately 20,000 partitioned droplets and can have up to 10,000,000 per reaction. The QuantStudio 12k dPCR performs digital PCR analysis on an OpenArray® plate which contains 64 reaction areas per subarray and 48 subarrays in total, equating to a total of 3072 reaction areas per array.

Droplet dPCR (ddPCR) is based on water-oil emulsion droplet technology. A sample is fractionated into a multitude of droplets (e.g. about 20,000) and PCR amplification of the template molecules occurs in each individual droplet. ddPCR technology uses reagents and workflows similar to those used for most standard TaqMan probe-based assays including droplet formation chemistry. Also, an intercalating dye, such as Evagreen, may be used. The massive sample reaction partitioning is a key aspect of the ddPCR technique. Non-spherical partitions (e.g. nanowells) actually have a larger area per sample volume than the same number of spherical partitions.

Typically, the accuracy and more importantly the precision of determination by dPCR may be improved by using a greater number of reaction areas. One may use approximately, 100 to 200, 200 to 300, 300 to 400, 700 or more reaction areas, which are used for determining the amount or concentration in question by PCR.

In a second step of the method of the first aspect, the distribution of the optical signals in each reaction area is determined.

For this, optical signals from various sub-areas of each reaction area are detected and determined. Preferably, the reaction area is subdivided into sub-areas for analysis and an optical signal is obtained for each sub-area, thereby the distribution of optical signals in the reaction area is determined. Subdivision of the reaction area into sub-areas may be done by gridding or rasterizing, wherein the reaction area is subdivided into a generally rectangular grid of pixels, or points of color. The image is a dot matrix data structure. A raster is technically characterized by the width and height of the pixels and by the number of pixels per reaction area. In the present invention, a value characterizing the optical signal for each sub-area is detected, determined and registered for further analysis.

Typically, optical signals are detected by registering photons, which produces a recordable output, usually as an electrical signal. The intensity of the electrical signal corresponds to the intensity of the optical signal. However, methods for detecting optical signals are well-known in the art.

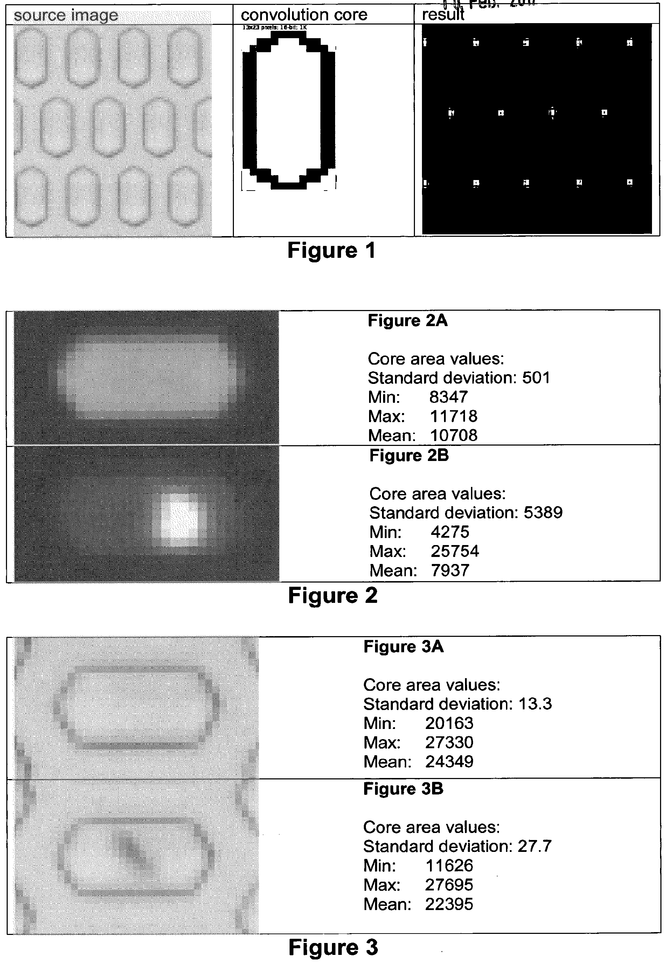

The optical signal may be determined in non-fluorescent bright- or dark- field mode or in fluorescent mode. Also in fluorescent mode, the value for the optical signal of the sub-areas is determined with a suitable image processing filter operation as for example the 2d-convolution of the image with a suitable filter core. This method is very efficient for the detection of a large number of e.g. pixel-wise relatively small objects. The result of the image processing filter operation is an image in which the center pixels of the partitions have peak values, these are detected then (see e.g. Figure 1).

As a third step, a reaction area is identified as invalid, if the optical signals in the reaction area determined in step b) are unequally distributed in the reaction area.

It is expected that the dPCR composition, which is usually a liquid, in a reaction area is equally distributed. Accordingly, it can be expected that the optical signals in the sub-areas of the reaction area are essentially identical. An unequal distribution of the signals hints at an artefact. The signals are unequally distributed, if there is at least one sub-area (preferably at least 3, more preferably at least 5, most preferably at least 10) having a significantly increased or decreased optical signal in comparison to the signals of the other sub-areas. In order to assess the distribution, a value for each sub-area is recorded. The sub-areas may be specified by consecutive numbers or letters, a XY-position of the area on the imaging system, or a position in a row of values and a value for the optical signal is assign to the sub-area. This process allows for identification of reaction areas on an array with their properties (e.g. intensity of the optical signal expressed as numeric value).

Thereafter, the distribution is assessed. This may be done by comparing the single values to each other. The distribution of the signals is unequal, if a single value differs significantly from any other value or the other values or if the difference between the value and any other value or the other values of the reaction area is above a threshold.

The person skilled in the art knows statistical procedures to assess whether two values are significantly different from each other such as Student's t-test or chi-square test. Furthermore, the skilled person knows how to select a suitable reference, such as a single value or group of values for (a) sub-area(s) of the reaction area. Alternatively, it may be a value determined previously, e.g. in a previous assay or a value provided by a third person, e.g. the manufacturer of laboratory equipment or a published value known from the art.

For artefact identification and invalidation of reaction areas, basically, there are different algorithms executed using the optical signals e.g. pixel belonging to a specific sub-area to determine the signal intensity distribution in the reaction area and therefore to determine an artefact and its type. Parameters such as the mean signal level, the median, the standard-deviation, the largest and lowest signal steps, also the shape of the distribution are calculated and determined. If filling of a partition is not appropriate, this can also be recognized by using threshold values for the signal and treated as an artefact, based on the parameter set.

With respect to the selection of a threshold it is noted that the person skilled in the art will be able to select a suitable threshold, based on his experience and the circumstance of the prevailing case (e.g. the assay or marker involved). The experience may also include previous similar or identical assays and the variation of optical signals recorded therein.

Alternatively or additionally, an average value or mean value for the optical signals and optionally a standard deviation of the values for the optical signals in the reaction area is determined. Accordingly, equal distribution may also be assessed by analyzing the mean, the standard deviation, the shape of distribution or the deviation of a single signal from the mean of signals. The reaction area may be identified as invalid, if the distribution of the optical signals in the reaction area is characterized by (i) a standard deviation above a threshold, (ii) an inappropriate shape of distribution, (iii) a deviation of a single signal from the mean of signals above a threshold and/or (iv) a mean of the signals deviating significantly from the expected. Examples for identifying optical artefacts are also shown in Figures 2 and 3. As can be seen in these Figures, the standard deviation is significantly increased in reaction areas with a dust artefact. These reaction areas show also an inappropriate shape of distribution. The deviation of the minimal and/or maximal signal from the mean of signals is significantly increased and the mean of the signals deviates from that measured in the reaction areas without artefact.

A suitable threshold level may be determined and derived from the typical standard deviations e.g. implicitly in the same array.

Additionally, sub-areas (e.g. pixels) surrounding the reaction area may be included in the assessment and invalidation of reaction areas. This may include the analysis of e.g. the average rim value or the STDEV of rim values.

If a reaction area is considered to be invalidated such as described in the previous section, a further artefact identification method can be applied by investigating the signal values for sub-areas environmental to the reaction area. By applying similar concept as described in the previous section, calculating the mean signal level, the median, the standard-deviation, the largest and lowest signal steps, also the shape of the distribution (shape) of the sub-areas assigned to this rim area, the certainty of artefact recognition can be enlarged, since most of the artefacts have larger lateral size than the reaction area dimensions.

In an exemplary embodiment a sensor is placed in focus position to the reaction areas of the array and an image is captured. After all, this captured image can be evaluated. Usually, the complete sensor e.g. camera pixel is read. Typically the optical signals are conferred into numeric values, wherein several values for the sub-areas are obtained.

For artefact identification and invalidation of the reaction area, the values are compared to each other. There are different procedures and algorithms which may be used in the analysis using the optical signals e.g. pixel belonging to a specific sub-area to determine the signal intensity distribution in the reaction area and therefore to determine an artefact and optionally its type. Parameters such as the mean signal level, the median, the standard-deviation, the largest and lowest signal steps, also the shape of the distribution (hexagon partition shape vs. e.g. a circular shape in the partition) are calculated and determined. If filling of reaction area is not appropriate, this can also be recognized by using threshold values for the optical signal and treated as an artefact, based on the parameter set.

As a fourth step, the reaction area identified as invalid is eliminated from calculating the amount or concentration of the nucleic acid of interest. "Eliminated" means that the reaction area is treated as not having been assessed and is ignored. Typically, neither the signal (positive or negative; "0" and "1", see above), nor the volume of the reaction area are considered when quantifying the amount or concentration of a nucleic acid of interest in the array of reaction areas.

In a second aspect, the present invention relates to a method for determining the amount or concentration of a nucleic acid of interest in a sample, the method comprising the steps of:
a) performing dPCR with a sample suspected of containing the nucleic acid of interest in each reaction area of an array of reaction areas;
b) determining the distribution of the optical signals in each reaction area;
c) identifying a reaction area as invalid, if the optical signals in the reaction area determined in step b) are unequally distributed in the reaction area; and
d) calculating the amount or concentration of the nucleic acid of interest based on the dPCR results of the reaction areas not identified as invalid in step c).

The sample may be any sample suspected of containing the nucleic acid in question, including a sample from a subject. A sample is a limited quantity of material which is intended to be identical to and represent a larger amount of that material(s). An act of obtaining a sample can be done by a person or automatically. Samples can be taken or provided for testing, analysis, inspection, investigation, demonstration, or trial use. Sometimes, sampling may be continuously ongoing. The sample may comprise or consist of a solid, a liquid or a gas; it may be material of some intermediate characteristics such as gel or sputum, tissue, organisms, or a combination of these. Preferably, the sample is liquid or a suspension which allows for easy distribution.

Even if a material sample is not countable as individual items, the quantity of the sample may still be describable in terms of its volume, mass, size, or other such dimensions. A solid sample can come in one or a few discrete pieces, or can be fragmented, granular, or powdered.

The sample in the present context is a quantity of material that is suspected of containing one or more nucleic acids that are to be detected or measured and quantified. As used herein, the term includes - without limitation - a specimen (e.g., a biopsy or medical specimen), a culture (e.g., microbiological culture) or an environmental sample such as water or soil. Samples may be from a subject, such as an animal or human, they may be fluid, solid (e.g., stool), a suspension or tissue. The term "sample from a subject" includes all biological fluids, excretions and tissues isolated from any given subject. Preferably, the subject is an animal, more preferably a mammal or still more preferably a human. The sample may be obtained from all of the various families of domestic animals, as well as feral or wild animals, including, but not limited to, such animals as ungulates, bear, fish, rodents, etc.

Examples of samples include, but are not limited to, cell or tissue cultures, blood, blood serum, blood plasma, needle aspirate, urine, semen, seminal fluid, seminal plasma, prostatic fluid, excreta, tears, saliva, sweat, biopsy, ascites, cerebrospinal fluid, pleural fluid, amniotic fluid, peritoneal fluid, interstitial fluid, sputum, milk, lymph, bronchial and other lavage samples, or tissue extract samples. The source of the sample may be solid tissue as from a fresh, frozen and/or preserved organ or tissue sample or biopsy or aspirate; or cells from any time in gestation or development of the subject.

The sample may contain compounds which are not naturally intermixed with the source of the sample in nature such as preservatives, anticoagulants, buffers, fixatives, nutrients, antibiotics, or the like.

If the sample is not ready or suitable for dPCR, further processing before being used in dPCR might be required. Usually, the samples need to be processed for dPCR by e.g. diluting the sample (to obtain a concentration of nucleic acids allowing for dPCR), removing disturbing components, adding reagents required for dPCR etc. The processing may comprise a multitude of different steps and techniques, which will depend on various aspects, including the nature of the sample, the type of nucleic acid of interest and the dPCR method used. Typically, the processing includes purification steps and/or dilution or concentration steps. Methods for purifying nucleic acids are well-known in the art and include - without limitation - homogenization, washing, centrifugation, extraction, etc. It might be necessary to preserve the sample, e.g. by disruption of the sample, by adding preservatives, by freezing or drying the sample. For disruption of the sample obtained, physical force (e.g. a polytron, grinding or freezing) or chemical methods (e.g. lysis of cells) may be used. A detergent or a chaotrope may be used for homogenization. Nucleic acids may be extracted by the use of acid phenol/chloroform, filters, glass particles or chromatography (e.g. with appropriate nucleic acids as binding partner). It might be necessary to store the sample at any time of the processing (at the beginning, during and/or at the end of the processing). For this it might be necessary or suitable to add an appropriate medium, such as a buffered saline. It might be required to remove contaminants and/or nucleic acids, which are not of interest or might be disturbing. Enzymes may be used for removal of contaminants (such as a DNase, an RNase and/or a proteinase) or protection of the nucleic acid of interest (such as a DNase inhibitor or an RNase inhibitor). For inactivation of enzymes a heating step might be appropriate. Removal agents may be used in order to remove undesired components such as divalent cations (Ca²⁺ and Mg²⁺). Washing steps might be required to exchange media.

As detailed above, for dPCR the nucleic acid of interest has to be present in an appropriate amount or concentration during the dPCR. Accordingly, appropriate dilution or concentration steps might be required. Dilution of nucleic acid is usually performed by adding a solvent (such as an appropriate medium for the steps to follow, e.g. a dPCR medium or dPCR buffer). It may be accompanied by washing steps, if e.g. removal of undesired components or concentration in order to obtain certain final concentrations should be intended or required. Concentration may be done by any enrichment procedure such as immunocapture, centrifugation, alcohol precipitation and the use of a binding matrix. After the processing, the sample is ready for dPCR, which is to follow in step b) of the method of the invention according to the second aspect.

As detailed above, the sample contains a nucleic acid of interest, the amount or concentration of which is to be determined in the method of the present invention. A nucleic acid is a biopolymer essential for all known forms of life. Therefore, nucleic acids may be used as indicator for a particular organism, but also e.g. in case of mutations or naturally occurring variants, as indicator for a disease. Nucleic acids, which include DNA (deoxyribonucleic acid) and RNA (ribonucleic acid), are made from monomers known as nucleotides. Each nucleotide has three components: a 5-carbon sugar, a phosphate group, and a nitrogenous base. If the sugar is deoxyribose, the polymer is DNA. If the sugar is ribose, the polymer is RNA. Nucleic acids are among the most important biological macromolecules. They are found in abundance in all living organisms, where they function in encoding, transmitting and expressing genetic information - in other words, information is conveyed through the nucleic acid sequence, or the order of nucleotides within a DNA or RNA molecule. Experimental studies of nucleic acids constitute a major part of modern biological and medical research, and form a foundation for genome and forensic science, as well as the biotechnology and pharmaceutical industries. Accordingly, the method of the invention may be used in any of these fields.

The nucleic acid may be indicative of a microorganism (such as a pathogen) and may be useful in the diagnosis of a disease, such as an infection. Infections may be caused by bacteria, viruses, fungi, and parasites or other nucleic acid containing objects. The pathogen may be exogenous (acquired from environmental or animal sources or from other persons) or endogenous (from the normal flora). Samples may be selected on the basis of signs and symptoms, should be representative of the disease process, and should be collected before administration of antimicrobial agents. The amount of the nucleic acid in the unprocessed sample may be indicative of the severity of the disease.

Alternatively, the nucleic acid may be indicative of a genetic disorder. A genetic disorder is a genetic problem caused by one or more abnormalities in the genome, especially a condition that is present from birth (congenital). Most genetic disorders are quite rare and affect one person in every several thousands or millions. Genetic disorders may or may not be heritable, i.e., passed down from the parents' genes. In non-heritable genetic disorders, defects may be caused by new mutations or changes to the DNA. In such cases, the defect will only be heritable if it occurs in the germ line. The same disease, such as some forms of cancer, may be caused by an inherited genetic condition in some people, by new mutations in other people, and mainly by environmental causes in still other people. Evidently, the amount of nucleic acid with mutation may be indicative of the disease state.

In the methods of the present invention, the amount or concentration of nucleic acids is determined. The amount of substance is a standards-defined quantity. The International System of Units (SI) defines the amount of substance to be proportional to the number of elementary entities present, with the inverse of the Avogadro constant as the proportionality constant (in units of mol). The SI unit for amount of substance is the mole. The mole is defined as the amount of substance that contains an equal number of elementary entities as there are atoms in 12 g of the isotope carbon-12. Therefore, the amount of substance of a sample is calculated as the sample mass divided by the molar mass of the substance. In the present context, the "amount" usually refers to the number of copies of the nucleic acid sequence of interest.

The concentration of a substance is the abundancy of a constituent divided by the total volume of a mixture. Several types of mathematical description can be distinguished: mass concentration, molar concentration, number concentration, and volume concentration. The term concentration can be applied to any kind of chemical mixture, but most frequently it refers to solutes and solvents in solutions. The molar (amount) concentration has variants such as normal concentration and osmotic concentration. Preferably, the concentration is the amount of a constituent given in numbers divided by the total volume of a mixture. In the context of the present invention, the concentration is usually "copies per volume".

Preferably, the sample provided is in a liquid, which eases further method steps.

As a next step, dPCR is performed with the sample in each reaction area of an array of reaction areas. In dPCR, the nucleic acid in question is amplified and detected, where a number of individual molecules are each isolated in a separate reaction area. Each reaction area (well, chamber, bead, emulsion, etc.) will have either a negative result, if no starting molecule is present, or a positive result for amplification and detection, if the targeted starting molecule is present. It is a technique where a limiting dilution of the sample is made across a number of separate PCR reactions such that part of the reactions have no template molecules and give a negative amplification result. In counting the number of positive PCR reactions at the reaction endpoint, the individual template molecules present in the original sample one-by-one are counted. PCR-based techniques have the additional advantage of only counting molecules that can be amplified, e.g., that are relevant to the massively parallel PCR step in the sequencing workflow. In the digital PCR-based methods, one distributes the nucleic acid to be analyzed into a number of different reaction areas (such as well, beads, emulsions, gel spots, chambers in a microfluidic device, etc.). It is important that some reaction areas, but not all, contain at least one molecule. Typically, each reaction area will contain one or zero molecules. In practice, there will be a random distribution of molecules into reaction areas such as wells. In the case where a percentage of reaction areas (e.g., 80%) is positive, a number of areas will contain one or more molecules (e.g., an average of 2.2 molecules per well). Statistical methods may be used to calculate the expected total number of molecules in the sample, based on the number of different reaction areas and the number of positives. This will result in a calculated amount or concentration of nucleic acids in the portion that was applied to the different reaction areas. A number of statistical methods based on sampling and probability can be used to arrive at this concentration. An example of such an analysis is given in Dube et al, arXiv:0809.1460v2 "Computation of Maximal Resolution of Copy Number Variation on a Nanofluidic Device using Digital PCR (2008)," found at arxiv.org, citation arXiv:0809.1460v2 [q-bio.GN], first uploaded on 8 September 2008. The publication provides a series of equations that may be used to estimate the concentration of molecules and statistical confidence interval based on the number of reaction areas used in a digital PCR array and the number of positive results. Another example of this type of calculation may be found in U.S. Patent Application US 2009/0239308 A1.

Usually, a Poisson distribution is used to predict the digital regime where only a single DNA amplicon will occur in a randomly discretized volume reactor to favor only one DNA amplicon of interest per reaction volume. In this way, the PCR amplified signal (e.g., a fluorescence) emitted by each reactor volume is the product of only one amplicon and is isolated from all other discrete reactor volumes. Quantification is then achieved by counting how many digital reactors emit an amplified fluorescent signal corresponding to an intercalating dye or a particular DNA polymerase probe sequence. Since each reactor volume is limited to no more than a single DNA strand in the digital regime, one can correctly assume that 100% of its amplified fluorescence signal comes from only that one DNA strand and corresponding primer and probe set. However, a very low-concentration regime is usually not favorable with respect to imprecision of result.

A number of methodologies for dPCR exist. For example, emulsion PCR has been used to prepare small beads with clonally amplified DNA - in essence, each bead contains one type of amplicon of dPCR. Fluorescent probe-based technologies, which can be performed on the PCR products "in situ" (i.e., in the same wells), are particularly well suited for this application. U.S. Pat. No. 6,440,705, contains a more detailed description of this amplification procedure. These amplifications may be carried out in an emulsion or gel, on a bead or in a multiwell plate.

dPCR also includes microfluidic-based technologies where channels and pumps are used to deliver molecules to a number of reaction areas. Suitable microfluidic devices are known in the art.

The dPCR is carried out essentially as a conventional PCR. The nucleic acids (reference or of interest) in a suitable medium are contacted with primers, probes and a thermostable polymerase (e.g. Taq polymerase) and thermocycled (cycles of repeated heating and cooling of the reaction for separation of strands and enzymatic replication. The medium usually contains deoxynucleotides, a buffer solution and ions (e.g. Mg²⁺).

The selectivity of PCR results from the use of primers that are complementary to the region targeted for amplification under specific thermal cycling conditions. The resulting amplification product is detected by use of a suitable probe, which is usually labelled, e.g. fluorescence-labelled. For mRNA-based PCR the RNA sample is first reverse-transcribed to complementary DNA (cDNA) with reverse transcriptase.

Typically, the PCR process consists of a series of temperature changes that are repeated 25 to 50 times. These cycles normally consist of three stages: the first, at around 95°C, allows the separation of the nucleic acid's double chain; the second, at a temperature of around 50 to 60°C, allows the binding of the primers with the DNA template; the third, at between 68 to 72°C, facilitates the polymerization carried out by the DNA polymerase. Due to the small size of the fragments the last step is usually omitted in this type of PCR as the enzyme is able to increase their number during the change between the alignment stage and the denaturing stage. In addition, a signal, e.g. fluorescence, is measured with a temperature of, for example, 80°C, in order to reduce the signal caused by the presence of primer dimers when a non-specific dye is used. The temperatures and the timings used depend on a wide variety of parameters, such as: the enzyme used to synthesize the DNA, the concentration of divalent ions and deoxyribonucleotides (dNTPs) in the reaction and the binding temperature of the primers.

In an embodiment, a dPCR method is provided that enables the unique ability to identify a greater number of fluorescent probe sequences (e.g., TaqMan probe sequences) by using multiple color, temporal, and intensity combinations to encode each unique probe sequence. Furthermore, less expensive non TaqMan-probe real-time PCR amplification indicators such as SYBR- or PicoGreen can be used to achieve multiplexed dPCR based on temporal cues alone, intensity cues alone, or intensity and temporal cues combined, thus distinguishing primer pairs at greater degrees with significant cost reductions. These can also be used to enhance controls and normalize results for greater accuracy if desired. The typical multiplexing limits from typical 5-plex qPCR can be increased to as much as 100-plex dPCR with limited spectral bands using fluorescent reporters.

Prior to, simultaneously with or after the dPCR, the distribution of the optical signals in each reaction area is determined. Thereafter, a reaction area is identified as invalid, if the optical signals in the reaction area determined in step c) are unequally distributed in the reaction area and the reaction area identified as invalid is eliminated from calculating the amount or concentration of the nucleic acid of interest. Further details of these steps are also given above and below.

In the methods of the present invention, the amount or concentration of nucleic acids is determined. The amount of substance is a standards-defined quantity. The International System of Units (SI) defines the amount of substance to be proportional to the number of elementary entities present, with the inverse of the Avogadro constant as the proportionality constant (in units of mol). The SI unit for amount of substance is the mole. The mole is defined as the amount of substance that contains an equal number of elementary entities as there are atoms in 12 g of the isotope carbon-12. Therefore, the amount of substance of a sample is calculated as the sample mass divided by the molar mass of the substance.

The concentration of a substance is the abundancy of a constituent divided by the total volume of a mixture. Several types of mathematical description can be distinguished: mass concentration, molar concentration, number concentration, and volume concentration. The term concentration can be applied to any kind of chemical mixture, but most frequently it refers to solutes and solvents in solutions. The molar (amount) concentration has variants such as normal concentration and osmotic concentration. Preferably, the concentration is the amount of a constituent given in numbers divided by the total volume of a mixture.

The nucleic acid of interest according to the present invention is any nucleic acid, the amount or concentration of which is to be determined. A nucleic acid is a biopolymer essential for all known forms of life. Therefore, nucleic acids may be used as indicator for a particular organism, but also e.g. in case of mutations or naturally occurring variants, as indicator for a disease. Nucleic acids, which include DNA (deoxyribonucleic acid) and RNA (ribonucleic acid), are made from monomers known as nucleotides. Each nucleotide has three components: a 5-carbon sugar, a phosphate group, and a nitrogenous base. If the sugar is deoxyribose, the polymer is DNA. If the sugar is ribose, the polymer is RNA. Nucleic acids are among the most important biological macromolecules. They are found in abundance in all living organisms, where they function in encoding, transmitting and expressing genetic information - in other words, information is conveyed through the nucleic acid sequence, or the order of nucleotides within a DNA or RNA molecule. Experimental studies of nucleic acids constitute a major part of modern biological and medical research, and form a foundation for genome and forensic science, as well as the biotechnology and pharmaceutical industries. Accordingly, the method of the invention may be used in any of these fields.

In a preferred embodiment of the methods of the invention, the reaction area is identified as invalid, if the distribution of the optical signals in the reaction area is characterized by (i) a standard deviation above a threshold, (ii) an inappropriate shape of distribution, (iii) a deviation of a single signal from the mean of signals above a threshold (iv) a mean of the signals deviating significantly from the expected. Further details on these embodiments are given above.

Even though, the method does not require the use of a marker or label and may be carried out in its absence, e.g. be only determining the reaction area in bright- or dark-field or detection method, the use of a marker is preferred. According, in a preferred embodiment, the optical signals are determined by using an optical marker, preferably an optically detectable fill control marker or an optically detectable PCR probe.

An optical marker is a marker, which is optically detectable. The marker may be any compound, which is already present in the dPCR mixture and has a function in dPCR, (e.g. an optically detectable PCR probe) or may be any compound added in order to identify the artefact. For example, the optical marker may be a fill control marker, which is also used in order to control the filling in each reaction area and optionally to determine the volume of the reaction area may based on the amount or concentration of fill control marker detected in each reaction area (see also European patent application EP-A1-3299470).

Accordingly, the optical marker may be added to each reaction area of an array of reaction areas used in dPCR (i) in order to reduce quantification errors caused by an optical artefact and optionally (ii) in order to reduce quantification errors caused by reaction volume deviations and/or in order to be used as an optically detectable PCR probe. The marker may be any optically detectable substance or composition optionally further allowing quantifying the reaction volume in each reaction area and/or detecting nucleic acids as a probe.

Accordingly, the marker may consist of or comprise any optically detectable label. The term "label" as used herein generally refers to any kind of substance or agent which can be used to visualize, detect, analyze and/or quantify the volume in a reaction area. A label may, for example, be a dye that renders the volume optically detectable and/or optically distinguishable. A label according to the present invention may include, but is not limited to, any colored (e.g. 2,4-dinitrophenol) or luminescent, preferably fluorescent, molecule or an absorbance marker (non-fluorescing or fluorescent) which can be detected and/or visualized by means of luminescence analysis such as fluorescein dyes including, but not limited to, carboxyfluorescein (FAM), 6-carboxy-4',5'-dichloro-2'7'-dimethoxyfluorescein (JOE), fluoresceinisothiocyanat (FITC), tetrachlorofluorescein (TET), and hexachlorofluorescein, rhodamine dyes such as, e.g., carboxy-X-rhodamine (ROX), Texas Red and tetramethylrhodamine (TAMRA), cyanine dyes such as pyrylium cyanine dyes, DY548, Quasar 570, or Cy3, Cy5, Alexa 568, and alike. Fluorescent labels are commercially available from diverse suppliers including, for example, Invitrogen™ (USA).

The choice of the label is typically determined by its physical properties (e.g. spectral properties), by the availability of equipment for detecting and by the label(s) used in the detection of the nucleic acids in the dPCR. Labels as well as their detection strategies are well known to the person skilled in the art.

The marker has any suitable uniform or equal distribution in each reaction area, e.g., in a flow stream, in a multiwell-plate, on a chip, in an array or field of view, among others, in order to also allow for identification of optical artefacts. In addition, the label may indicate the volume of each reaction area. Methods for determining the amount or concentration of a marker/label will depend from the marker used and are well known in the art. Preferably, a fluorescence marker is used, which can be detected and/or quantified by means of fluorescence.

For example, the signal may be a fluorescence signal. If two or more different fluorescence signals are measured from each reaction area (one for PCR and one for identifying quantification errors and optionally as fill control marker), the signals may, for example, be detected at distinct wavelengths or wavebands. Alternatively, the fluorescence signals may be measured at the same wavelength/waveband after excitation with different wavelengths or wavebands of light (e.g., excitation at different times or at different positions), among others. Two or more fluorescence signals may be detected via respective distinct fluorophores.

In some embodiments, the marker may be not coupled to an amplification reaction and thus serves as a passive reference. In some embodiments, the marker may be additionally used as a control signal detected from a control amplification reaction. The control amplification reaction may measure amplification of an exogenous or endogenous template. Preferably, the label is stable during the method, not subject to bleaching, independent from the amplification reaction and/or temperature invariant.

Evidently, the optical marker can be added to the reaction area at different times, also depending on the assay design, the optical marker used and its properties. In general, the marker may be added to the reaction areas before or after dPCR is carried out.

For example, the marker can be present in the reaction area before the reagents necessary to carry out the dPCR are added. In one example, the may be distributed to the array, when the array is produced or manufactured.

Alternatively, the marker may be distributed to the reaction areas along with the dPCR reagents. This is particularly suitable if the amount or concentration of the marker is measured. In the methods of the invention, the marker, especially the fill control marker, is preferably added to the PCR reaction mix before being distributed to the reaction areas.

If the marker is used in the reduction of quantification errors caused by an optical artefact and in the determination of the volume in the array, it is referred to as the fill control marker. It may be further use as normalizing factor for the signal obtained for the dPCR and/or to eliminate other invalid samples from the determination. Reaction volume deviations may result in non-uniform volumes in the reaction areas. The width of the size distribution of the areas affects the count rate and hence the accuracy of the calculated nucleic acid concentration. This quantification error is negligible at low copy numbers per area, but may become large at high copy numbers per area. This effect can be illustrated by a thought experiment whereby one area becomes very large while all others shrink towards zero. In this case the positive count would obviously tend towards one, resulting in an extreme under-quantification. Alternatively or additionally, the reaction volume deviations may be due to the fact that the average reaction area volume differs from the intended or expected one, e.g. as predetermined by a calibration value. Both in droplet-based and array-based dPCR systems, uncertainties in the average partition volume were found to be a major source of accuracy errors in current systems (Dong et al., 2015, Sci. Rep. 5, 13174 and Dong et al., 2014, Anal. Bioanal. Chem. 406, 1701-1712).

Average reaction area volumes may change due to changes in reaction mix composition (in the case of dPCR arrays detergents have an effect on the meniscus forming at the interface of the reaction mix and the sealing fluid), changes in reaction area depths caused by the production process of the arrays (e.g. changes of the molding tools used for producing the arrays may be associated with geometric changes of the reaction areas) or changes in filling degree of the reaction area due to the filling speed.

Volume pertains to the three-dimensional space that is occupied by an object (i.e. solid, liquid, gas, or plasma). Volumes may be calculated based on the dimensions, e.g. the length, width, and height of the occupied space by an object. They are usually expressed in SI units, e.g. cubic centimeter (cm³), cubic meter (m³), liter (L), milliliter (mL), etc. Details on the determination of volumes are also given in European patent application EP-A1 -3299470.

The amount or concentration of the nucleic acid of interest may be calculated as number of nucleic acid as determined by dPCR in a or per volume, wherein the volume is the sum of the reaction volumes as determined with the use of the fill control marker. The fill control marker is used for the determination of the true volume in each reaction area and thus for the determination of the total and true volume analyzed by dPCR. It is evident that knowing the correct volume is important for determining the correct amount of the nucleic acid in question or its concentration. The total volume is the sum of the volumes in the reaction areas. The amount of nucleic acids in numbers is obtained by dPCR. The concentration of the nucleic acid is usually given as number of nucleic acids / volume, e.g. µl. Further details on the method and calculation of the amount or concentration of the nucleic acid of interest based on the dPCR results of the reaction areas are given in European patent application EP-A1 -3299470.

The accuracy and precision of the quantification with dPCR can be further increased if all reaction areas are classified as valid positive, valid negative or invalid reaction areas:
- A valid positive reaction area is properly filled with reaction mix and generates a positive PCR signal after amplification.
- A valid negative reaction area is properly filled with reaction mix and generates no PCR signal after amplification.
- An invalid reaction area is not (or insufficiently) filled with reaction mix or is eliminated because of an optical artefact.

As detailed above, it is important to identify invalid reaction areas. A reaction area may be invalid, because the filling or distribution process prior to dPCR may result in reaction areas of the array with empty or insufficiently filled reaction areas. If they are misinterpreted as filled wells without nucleic acid (negatives), the sampled volume V is overestimated and the concentration underestimated. These reaction areas can be identified using the fill control marker, e.g. if the signal of the fill control marker is below a lower threshold, and eliminated from the calculation. Alternatively or additionally, if the signal level of the fill control marker from a particular reaction area is above a predefined threshold value, this reaction area may be discarded from the calculation of target concentration. Usually, a reaction area cannot be filled beyond its maximum height. Larger signal levels indicate artefacts that may originate from fluorescing dust particles and other contaminations.

In a preferred embodiment of the present invention, the distribution is determined by raster imaging of each reaction area, particularly wherein each raster representing an optical signal consists of a constant number of pixels, especially one pixel, of an optical device, particularly a camera and/or wherein the distribution is characterized by the mean of the optical signals, the median, the standard-deviation, the largest and lowest signal step and/or the shape of the distribution. Further details on this embodiment are given above.

In a preferred embodiment of the invention, the optical signals are determined by a non-fluorescent bright- or dark-field or fluorescence detection method.

Bright-field microscopy is the simplest of all the optical microscopy illumination techniques. Sample illumination is transmitted (i.e., illuminated from below and observed from above) white light and contrast in the sample is caused by absorbance of some of the transmitted light in dense areas of the sample. Bright-field microscopy is the simplest of a range of techniques used for illumination of samples in light microscopes and its simplicity makes it a popular technique. The typical appearance of a bright-field microscopy image is a dark sample on a bright background, hence the name.

Dark field microscopy (dark ground microscopy) describes microscopy methods, in both light and electron microscopy, which exclude the unscattered beam from the image. As a result, the field around the specimen (i.e., where there is no specimen to scatter the beam) is generally dark. Dark field describes an illumination technique used to enhance the contrast in unstained samples. It works by illuminating the sample with light that will not be collected by the objective lens, and thus will not form part of the image. This produces the classic appearance of a dark, almost black, background with bright objects on it.

Fluorescence is the emission of light by a substance that has absorbed light or other electromagnetic radiation. It is a form of luminescence. In most cases, the emitted light has a longer wavelength, and therefore lower energy, than the absorbed radiation. The most striking example of fluorescence occurs when the absorbed radiation is in the ultraviolet region of the spectrum, and thus invisible to the human eye, while the emitted light is in the visible region, which gives the fluorescent substance a distinct color that can only be seen when exposed to UV light. Fluorescent materials cease to glow immediately when the radiation source stops.

In a preferred embodiment of the present invention, the optical artefact is due to dust, a scratch, fluid splash, hair, fiber, fingerprint, incorrect filling of a reaction area and/or a defect in the array structure. Further details on these terms are given above.

In another preferred embodiment of the methods of the invention, the optical marker is a fluorescence marker or an absorbance marker.

A wide range of fluorescence markers can be used as fill control markers in accordance with the present invention. Each fluorescence marker has a characteristic peak excitation and emission wavelength, and the emission spectra often overlap. Consequently, the combination of fluorescence markers used for dPCR and fill control depends on the wavelength of the lamp(s) or laser(s) used to excite the fluorochromes, the detectors available and the properties of the markers.

Exemplary fluorescent dyes that may be detected using system 6010 include a fluorescein derivative, such as carboxyfluorescein (FAM), and a PULSAR 650 dye (a derivative of Ru(bpy)3). FAM has a relatively small Stokes shift, while PULSAR 650 dye has a very large Stokes shift. Both FAM and PULSAR 650 dye may be excited with light of approximately 460-480 nm. FAM emits light with a maximum of about 520 nm (and not substantially at 650 nm), while PULSAR 650 dye emits light with a maximum of about 650 nm (and not substantially at 520 nm). Carboxyfluorescein may be paired in a probe with, for example, BLACK HOLE Quencher™ 1 dye, and PULSAR 650 dye may be paired in a probe with, for example, BLACK HOLE Quencher™2 dye.

More preferably, the fluorescence and/or absorbance properties of the optical marker should be different from that of the one or more dPCR probe(s). This is of advantage in order to allow to conveniently distinguish the optical marker and the fluorescent dPCR probe(s). However, the detection can be further simplified, if the optical marker has an excitation wavelength or an emission wavelength identical to a target probe fluorescence marker used in the dPCR. This way the optical marker does not reduce the color multiplexing capability of the detection system. As an example, the system may have 4 excitation and 4 emission channels. The large Stokes shift dye is excited by excitation wavelength 1 and the emission is collected from emission channel 4, which are also used for dPCR.

In another more preferred embodiment, the optical marker has a Stokes-shift of at least 100 nm, preferably at least 150 nm. Stokes shift is the difference (in wavelength) between positions of the band maxima of the absorption and emission spectra of the same electronic transition. When a molecule absorbs a photon, it gains energy and enters an excited state. As a result it emits a photon, thus losing its energy. When the emitted photon has less energy than the absorbed photon, this energy difference is the Stokes shift. A larger Stokes shift eliminates spectral overlap between absorption and emission and allows detection of fluorescence while reducing interference. The main advantage is that a large Stokes shift dye can be used together with other dyes having either a similar excitation or emission spectrum. Since one of both spectra, however, does not overlap between the small and the large Stokes shift dyes, spectral crosstalk is small.

Preferred optical marker include ATTO 430LS and ATTO 490LS (available from ATTO-TEC GmbH, Siegen, DE), especially ATTO 490LS. Both show good solubility in water and a Stokes-Shift of more than 100 nm, which particularly useful in methods with multiple fluorescence markers, as the high Stokes Shift minimizes an overlap of signals during detection. ATTO 490LS has the excitation wavelength of FAM and the emission wavelength of Cy5. If combined with FAM and Cy5, no additional filters for measuring ATTO 490LS are required.

In another preferred embodiment of the methods of the present invention according to the first and second aspect the nucleic acid of interest is a nucleic acid selected from the group consisting of DNA, cDNA, RNA and a mixture thereof, or is any other type of nucleic acid.

As detailed above, the nucleic acid of interest may be any nucleic acid suitable for dPCR. The nucleic acid has to have a suitable length. It may contain non nucleic acid components. It may be naturally occurring, chemically synthesized or biotechnologically engineered. Preferably, the nucleic acid is selected from the group consisting of DNA, cDNA, RNA and a mixture thereof.

The methods of the invention are of particular interest in the medical field such as in diagnosis or in therapeutic monitoring and may be used in order to detect and/or quantify a nucleic acid of interest indicative of a specific microorganism, cell, virus, bacterium, fungus, mammal species, genetic status or a disease. In accordance with this, the methods may be used in the detection of a pathogen. A pathogen has the potential to cause a disease. Typically pathogen is used to describe an infectious agent such as a virus, bacterium, prion, a fungus, or even another microorganism. Of cause, the methods of the invention may also be used to detect non-pathogenic microorganisms.

Exemplary pathogens include without limitation:
- Bacterial: Streptococcus, Staphylococcus, Pseudomonas, Burkholderia, Mycobacterium, Chlamydophila, Ehrlichia, Rickettsia, Salmonella, Neisseria, Brucella, Mycobacterium, Nocardia, Listeria, Francisella, Legionella, and Yersinia
- Viral: Adenovirus, Herpes simplex, Varicella-zoster virus, Cytomegalovirus Papillomavirus, Hepatitis B virus Hepatitis C virus, Hepatitis E virus, Poliovirus, Yellow fever virus, Dengue virus, West Nile virus, TBE virus, HIV, Influenza virus, Lassa virus, Rotavirus and Ebola virus
- Fungal: Candida, Aspergillus, Cryptococcus, Histoplasma, Pneumocystis and Stachybotrys
- Parasites: protozoan parasites, helminth parasites and arthropod parasites

In still another preferred embodiment of the methods of the present invention according to the second aspect the sample has been obtained from a cell culture or a source suspected of being contaminated, particularly a body fluid, blood, blood plasma, blood serum, urine, bile, cerebrospinal fluid, a swab, a clinical specimen, an organ sample or a tissue sample or a subject, particularly a human, an animal or a plant, especially a human.

As detailed above, "sample" means a quantity of material that is suspected of containing a nucleic acid of interest that is to be quantified. As used herein, the term includes a specimen (e.g., a biopsy or medical specimen) or a culture (e.g., microbiological culture). Samples may be from a plant or animal, including human, it may be fluid, solid (e.g., stool) or tissue. Samples may include materials taken from a patient including, but not limited to cultures, blood, saliva, cerebral spinal fluid, pleural fluid, milk, lymph, sputum, semen, needle aspirates, and the like. The sample may be obtained from all of the various families of domestic animals, as well as feral or wild animals, including, but not limited to, such animals as ungulates, bear, fish, rodents, etc. In regard to a human sample or "tissue sample" or "patient sample" or "patient cell or tissue sample" or "specimen," each means a collection of similar cells or biological or biochemical compounds obtained from a tissue of a subject or patient. The source of the tissue sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; or cells from any time in gestation or development of the subject. The tissue sample may contain compounds which are not naturally intermixed with the tissue in nature such as preservatives, anticoagulants, buffers, fixatives, nutrients, antibiotics, or the like.

In a preferred embodiment of the methods of the present invention according to the first and second aspect the dPCR is carried out identically in at least 100 reaction areas, particularly at least 1,000 reaction areas, especially at least 5,000 reaction areas. In a preferred embodiment of the methods of the present invention according to the first and second aspect the dPCR is carried out identically in at most 1,000,000 reaction areas, particularly at most 100,000 reaction areas, especially at most 50,000, preferably at most 10,000 reaction areas.

Preferably, the dPCR involves the use of one or more fluorescent dPCR probes in order to detect one or more nucleic acid(s) of interest, particularly in combination with a quencher or as molecular beacon or as a hydrolysis probe.

In PCR applications (such as Real Time PCR) fluorescence is often used to detect amplification products. It is usually carried out in a thermal cycler with the capacity to illuminate each sample with a beam of light of at least one specified wavelength and detect the fluorescence emitted by the excited fluorophore. The thermal cycler is also able to rapidly heat and chill samples, thereby taking advantage of the physicochemical properties of the nucleic acids and DNA polymerase.

The dPCR may involve the use of one or more fluorescent probes in order to detect the nucleic acid of interest and/or the reference nucleic acid, particularly in combination with a quencher or as molecular beacon or as a hydrolysis probe.

Often Fluorescence Resonance Energy Transfer (FRET) is detected in qPCR. FRET is a technique for measuring interactions between two molecules, in the present case two probes. In this technique, two different fluorescent molecules (fluorophores or labels) are genetically fused to a pair of probes suitable for the detection of a nucleic acid. The principle of FRET is based on the combined characteristics of the two labels. If a label is excited with a light of a particular wavelength (absorption frequency) its re-emits that energy at a different wavelength (the emission frequency). In FRET the first label is excited which in turn emits light having the emission frequency. If the emission peak of the first label (donor) overlaps with the excitation peak of the second label (acceptor), proximity of the two labels can be determined, since the first label transfers energy to the second label and the second label emits light at its own emission frequency. The net result is that the donor emits less energy than it normally would (since some of the energy it would radiate as light gets transferred to the acceptor instead), while the acceptor emits more light energy at its excitation frequency (because it is getting extra energy input from the donor fluorophore). Also the combination of a fluorescent dye with a quencher may be used. If the quencher is in proximity to the fluorescent dye, the emission of fluorescence is omitted. If the fluorescent moiety becomes separated from the quencher, the emission of the first fluorescent moiety can be detected after excitation with light of a suitable wavelength. Molecular beacons are hairpin shaped probes with an internally quenched fluorophore whose fluorescence is restored when they bind to a target nucleic acid sequence. If the nucleic acid to be detected is complementary to the strand in the loop, the duplex formed between the nucleic acid and the loop is more stable than that of the stem because the former duplex involves more base pairs. This causes the separation of the fluorophore and the quencher. Once the fluorophore is distanced from the quencher, illumination of the hybrid with light results in the fluorescent emission. The presence of the emission reports that the event of hybridization has occurred and hence the target nucleic acid sequence is present in the test sample. Hydrolysis probes consist of a fluorophore covalently attached to the 5'-end of the oligonucleotide probe and a quencher at the 3'-end. As long as the fluorophore and the quencher are in proximity, quenching inhibits any fluorescence signals. The probes are designed such that they anneal within a DNA region amplified by a specific set of primers. As the polymerase extends the primer and synthesizes the nascent strand, the 5' to 3' exonuclease activity of the polymerase degrades the probe that has annealed to the template. Degradation of the probe releases the fluorophore from it and breaks the close proximity to the quencher, thus relieving the quenching effect and allowing fluorescence of the fluorophore. Hence, fluorescence detected is indicative of the presence of the nucleic acid in question.

Representative donor fluorescent moieties that can be used with various acceptor fluorescent moieties in FRET technology include fluorescein, Lucifer Yellow, B-phyco-erythrin, 9-acridineisothiocyanate, Lucifer Yellow VS, 4-acetamido-4'-isothiocyanatostilbene-2,2'-disulfonic acid, 7-diethylamino-3-(4'-isothiocyanatephenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC-Red 610, LC -Red 640, LC-Red 670, LC - Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium). Donor and acceptor fluorescent moieties can be obtained, for example, from Molecular Probes (Junction City, OR) or Sigma Chemical Co. (St. Louis, MO).

Preferably, the fluorescent probe comprises fluorescein, rhodamine and/or cyanine. For example, the donor fluorescent moiety may be fluorescein and/or the acceptor fluorescent moiety may be selected from the group consisting of LC-Red 610, LC-Red 640, LC-Red 670, LC-Red 705, Cy5, and Cy5.5, preferably LC-Red 610 or LC-Red 640. More preferably the donor fluorescent moiety is fluorescein and the acceptor fluorescent moiety is LC-Red 640 or LC-Red 610.

Several different fluorophores (e.g. 6-carboxyfluorescein, acronym: *FAM,* or tetrachlorofluorescein, acronym: TET) and quenchers (e.g. tetramethylrhodamine, acronym: TAMRA) are available.

The definitions, and examples made in the context of the methods of the first aspect of the invention also apply to those of the second aspect and vice versa.

Unless defined otherwise, all technical and scientific terms and any acronyms used herein have the same meanings as commonly understood by one of ordinary skill in the art in the field of the invention. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1 -56081 -569-8).

The invention is not limited to the particular methodology, protocols, and reagents described herein because they may vary. Although any methods and materials similar or equivalent to those described herein can be used in the practice of the present invention, the preferred methods, and materials are described herein. Further, the terminology used herein is for the purpose of describing particular embodiments only and is not intended to limit the scope of the present invention.

As used herein and in the appended claims, the singular forms "a", "an", and "the" include plural reference unless the context clearly dictates otherwise. Similarly, the words "comprise", "contain" and "encompass" are to be interpreted inclusively rather than exclusively. Similarly, the word "or" is intended to include "and" unless the context clearly indicates otherwise. The term "plurality" refers to two or more.

The Figures are intended to illustrate various embodiments of the invention. As such, the specific modifications discussed are not to be construed as limitations on the scope of the invention. It will be apparent to the person skilled in the art that various equivalents, changes, and modifications may be made without departing from the scope of the invention, which is defined by the claims.

### FIGURES

- **Figure 1**: illustrates steps from source image to the image containing the partitions center with peak pixels
- **Figure 2**: illustrates artefact removal on the dark-field fluorescence image. Figures 2A and 2B show a reaction area with unquenched dye (normal shape) and a reaction area with quenched dye and fluorescent dust artefact, respectively.
- **Figure 3**: illustrates artefact removal on the bright-field non fluorescence. Figures 3A and 3B show a reaction area without artefact (normal shape) and a reaction area with dust artefact, respectively. The core area consists of all pixels inside the partition borders which are not shaded by the partition-border. Artefacts are best discriminated by deviations of the standard deviation or the minimum value.

## Claims

1. A method for reducing quantification errors caused by an optical artefact in digital polymerase chain reaction (dPCR), wherein the amount or concentration of a nucleic acid of interest is quantified in an array of reaction areas, the method comprising
a) providing an array of reaction areas used in dPCR;
b) determining the distribution of the optical signals in each reaction area;
c) identifying a reaction area as invalid, if the optical signals in the reaction area determined in step b) are unequally distributed in the reaction area; and
d) eliminating the reaction area identified as invalid from calculating the amount or concentration of the nucleic acid of interest.

2. A method for determining the amount or concentration of a nucleic acid of interest in a sample, the method comprising the steps of:
a) performing dPCR with a sample suspected of containing the nucleic acid of interest in each reaction area of an array of reaction areas;
b) determining the distribution of the optical signals in each reaction area;
c) identifying a reaction area as invalid, if the optical signals in the reaction area determined in step b) are unequally distributed in the reaction area; and
d) calculating the amount or concentration of the nucleic acid of interest based on the dPCR results of the reaction areas not identified as invalid in step c).

3. The method of claim 1 or 2, wherein the reaction area is identified as invalid, if the distribution of the optical signals in the reaction area is **characterized by** (i) a standard deviation above a threshold, (ii) an inappropriate shape of distribution, (iii) a deviation of a single signal from the mean of signals above a threshold and/or (iv) a mean of the signals deviating significantly from the expected.

4. The method of any of claims 1 to 3, wherein the optical signals are determined by using an optical marker, preferably an optically detectable fill control marker or an optically detectable PCR probe.

5. The method of any of claims 1 to 4, wherein the distribution is determined by raster imaging of each reaction area, particularly wherein each raster representing an optical signal consists of a constant number of pixels, especially one pixel, of an optical device, particularly a camera and/or wherein the distribution is **characterized by** the mean of the optical signals, the median, the standard-deviation, the largest and lowest signal step and/or the shape of the distribution.

6. The method of any of claims 1 to 5, wherein the optical signals are determined by a non-fluorescent bright- or dark-field or fluorescence detection method.

7. The method of any of claims 4 to 6, wherein the optical artefact is due to dust, a scratch, fluid splash, hair, fiber, fingerprint, incorrect filling of a reaction area and/or a defect in the array structure.

8. The method of any of claims 4 to 7, wherein the optical marker is a fluorescence marker.

9. The method of claim 8, wherein the optical marker
- has fluorescence and/or absorbance properties different from that of the one or more dPCR probe(s); and/or
- has a Stokes-shift of at least 100 nm, preferably at least 150 nm; and/or
- is ATTO 430 LS or ATTO 490 LS, preferably ATTO 490 LS; and/or
- has an excitation wavelength or an emission wavelength identical to a target probe fluorescence marker used in the dPCR.

10. The method of any of claims 1 to 9, wherein the nucleic acid of interest
- is a nucleic acid selected from the group consisting of DNA, cDNA, RNA and a mixture thereof; and/or
- is indicative of a microorganism, a cell, a virus, a bacterium, a fungus, a mammal species, a genetic status or a disease.

11. The method of any of claims 2 to 10, wherein the sample has been obtained from a cell culture or a source suspected of being contaminated, particularly a body fluid, blood, blood plasma, blood serum, urine, bile, cerebrospinal fluid, a swab, a clinical specimen, an organ sample and a tissue sample or a subject, particularly a human, an animal and a plant, especially a human.

12. The method of any of claims 1 to 11, wherein the reaction area is a miniaturized chamber of a microarray or a nanoarray, a chamber of a microfluidic device, a microwell or a nanowell, on a chip, in a capillary, on a nucleic acid binding surface or on a bead, especially in a microarray or on a chip.

13. The method of any of claims 1 to 12, wherein the array of reaction areas comprises
- at least 100 reaction areas, particularly at least 1,000 reaction areas, especially at least 5,000 reaction areas; and/or
- at most 1,000,000 reaction areas, particularly at most 100,000 reaction areas, especially at most 50,000, preferably at most 10,000 reaction areas.

14. The method of any of claims 1 to 13, wherein the dPCR involves the use of one or more fluorescent dPCR probes in order to detect one or more nucleic acid(s) of interest, particularly in combination with a quencher or as molecular beacon or as a hydrolysis probe and/or particularly wherein the fluorescent dPCR probe comprises fluorescein, rhodamine and/or cyanine.

## Patentansprüche

1. Verfahren zum Reduzieren von Quantifizierungsfehlern, die durch ein optisches Artefakt in der digitalen Polymerase-Kettenreaktion (dPCR) verursacht werden, wobei die Menge oder Konzentration einer interessierenden Nukleinsäure in einer Anordnung von Reaktionsbereichen quantifiziert wird, wobei das Verfahren umfasst
a) Bereitstellen einer Anordnung von Reaktionsbereichen, die in dPCR verwendet werden;
b) Bestimmen der Verteilung der optischen Signale in jedem Reaktionsbereich;
c) Identifizieren eines Reaktionsbereichs als ungültig, wenn die optischen Signale in dem in Schritt b) bestimmten Reaktionsbereich ungleichmäßig in dem Reaktionsbereich verteilt sind; und
d) Eliminieren des als ungültig identifizierten Reaktionsbereichs aus der Berechnung der Menge oder Konzentration der interessierenden Nukleinsäure.

2. Verfahren zum Bestimmen der Menge oder Konzentration einer interessierenden Nukleinsäure in einer Probe, wobei das Verfahren die folgenden Schritte umfasst:
a) Durchführen der dPCR mit einer Probe, bei der der Verdacht besteht, dass sie die interessierende Nukleinsäure enthält, in jedem Reaktionsbereich einer Anordnung von Reaktionsbereichen;
b) Bestimmen der Verteilung der optischen Signale in jedem Reaktionsbereich;
c) Identifizieren eines Reaktionsbereichs als ungültig, wenn die optischen Signale in dem in Schritt b) bestimmten Reaktionsbereich ungleichmäßig in dem Reaktionsbereich verteilt sind; und
d) Berechnen der Menge oder Konzentration der interessierenden Nukleinsäure basierend auf den dPCR-Ergebnissen der Reaktionsbereiche, die in Schritt c) nicht als ungültig identifiziert wurden.

3. Verfahren nach Anspruch 1 oder 2, wobei der Reaktionsbereich als ungültig identifiziert wird, wenn die Verteilung der optischen Signale in dem Reaktionsbereich **gekennzeichnet ist durch** (i) eine Standardabweichung oberhalb eines Schwellenwerts, (ii) eine ungeeignete Verteilungsform, (iii) eine Abweichung eines einzelnen Signals vom Mittelwert der Signale oberhalb eines Schwellenwerts und/oder (iv) einen Mittelwert der Signale, der signifikant von dem erwarteten abweicht.

4. Verfahren nach einem der Ansprüche 1 bis 3, wobei die optischen Signale unter Verwendung eines optischen Markers, vorzugsweise eines optisch detektierbaren Füllkontrollmarkers oder einer optisch detektierbaren PCR-Sonde, bestimmt werden.

5. Verfahren nach einem der Ansprüche 1 bis 4, wobei die Verteilung durch Rasterabbildung jedes Reaktionsbereichs bestimmt wird, insbesondere wobei jedes Raster, das ein optisches Signal darstellt, aus einer konstanten Anzahl von Pixeln, insbesondere einem Pixel, einer optischen Vorrichtung, insbesondere einer Kamera, besteht und/oder wobei die Verteilung durch den Mittelwert der optischen Signale, den Median, die Standardabweichung, den größten und niedrigsten Signalschritt und/oder die Form der Verteilung gekennzeichnet ist.

6. Verfahren nach einem der Ansprüche 1 bis 5, wobei die optischen Signale durch ein nicht fluoreszierendes Hell- oder Dunkelfeld- oder Fluoreszenzdetektionsverfahren bestimmt werden.

7. Verfahren nach einem der Ansprüche 4 bis 6, wobei das optische Artefakt auf Staub, einen Kratzer, einen Flüssigkeitsspritzer, ein Haar, eine Fasern, einen Fingerabdruck, falsches Füllen eines Reaktionsbereichs und/oder einen Defekt in der Matrixstruktur zurückzuführen ist.

8. Verfahren nach einem der Ansprüche 4 bis 7, wobei der optische Marker ein Fluoreszenzmarker ist.

9. Verfahren nach Anspruch 8, wobei der optische Marker
- Fluoreszenz- und/oder Absorptionseigenschaften aufweist, die sich von denen der einen oder mehreren dPCR-Sonde(n) unterscheiden; und/oder
- eine Stokes-Verschiebung von mindestens 100 nm, vorzugsweise mindestens 150 nm aufweist; und/oder
- ATTO 430 LS oder ATTO 490 LS, vorzugsweise ATTO 490 LS ist; und/oder
- eine Anregungswellenlänge oder eine Emissionswellenlänge aufweist, die mit einem in der dPCR verwendeten Fluoreszenzmarker der Zielsonde identisch ist.

10. Verfahren nach einem der Ansprüche 1 bis 9, wobei die interessierende Nukleinsäure
- eine Nukleinsäure ist, ausgewählt aus der Gruppe bestehend aus DNA, cDNA, RNA und einer Mischung davon; und/oder
- ein Indikator für einen Mikroorganismus, eine Zelle, ein Virus, ein Bakterium, einen Pilz, eine Säugetierart, einen genetischen Status oder eine Krankheit ist.

11. Verfahren nach einem der Ansprüche 2 bis 10, wobei die Probe aus einer Zellkultur oder einer Quelle erhalten wurde, von der vermutet wird, dass sie kontaminiert ist, insbesondere einer Körperflüssigkeit, Blut, Blutplasma, Blutserum, Urin, Galle, Liquor, einem Tupfer, einer klinischen Probe, einer Organprobe und einer Gewebeprobe oder einem Subjekt, insbesondere einem Menschen, einem Tier und einer Pflanze, besonders einem Menschen.

12. Verfahren nach einem der Ansprüche 1 bis 11, wobei der Reaktionsbereich eine miniaturisierte Kammer eines Mikroarrays oder eines Nanoarrays, eine Kammer einer Mikrofluidikvorrichtung, ein Mikrowell oder ein Nanowell, auf einem Chip, in einer Kapillare, auf einer Nukleinsäure-bindenden Oberfläche oder auf einem Bead ist, besonders in einem Mikroarray oder auf einem Chip.

13. Verfahren nach einem der Ansprüche 1 bis 12, wobei die Anordnung der Reaktionsbereiche umfasst
- mindestens 100 Reaktionsbereiche, insbesondere mindestens 1.000 Reaktionsbereiche, besonders mindestens 5.000 Reaktionsbereiche; und/oder
- höchstens 1.000.000 Reaktionsbereiche, insbesondere höchstens 100.000 Reaktionsbereiche, besonders höchstens 50.000, vorzugsweise höchstens 10.000 Reaktionsbereiche.

14. Verfahren nach einem der Ansprüche 1 bis 13, wobei die dPCR die Verwendung einer oder mehrerer fluoreszierender dPCR-Sonden beinhaltet, um eine oder mehrere interessierende Nukleinsäure(n) nachzuweisen, insbesondere in Kombination mit einem Quencher oder als Molekularbeacon oder als Hydrolysesonde und/oder insbesondere wobei die fluoreszierende dPCR-Sonde Fluoreszein, Rhodamin und/oder Cyanin umfasst.

## Revendications

1. Procédé de réduction des erreurs de quantification causées par un artéfact optique dans une réaction en chaîne numérique de la polymérase (dPCR), la quantité ou la concentration d'un acide nucléique d'intérêt étant quantifiée dans un réseau de zones réactionnelles, le procédé comprenant
a) la fourniture d'un réseau de zones réactionnelles utilisé dans la dPCR ;
b) la détermination de la distribution des signaux optiques dans chaque zone réactionnelle ;
c) l'identification d'une zone réactionnelle comme invalide, si les signaux optiques dans la zone réactionnelle déterminés dans l'étape b) sont distribués de manière inégale dans la zone réactionnelle ; et
d) l'élimination de la zone réactionnelle identifiée comme invalide à partir du calcul de la quantité ou de la concentration de l'acide nucléique d'intérêt.

2. Procédé de détermination de la quantité ou de la concentration d'un acide nucléique d'intérêt dans un échantillon, le procédé comprenant les étapes de :
a) réalisation de dPCR avec un échantillon suspecté de contenir l'acide nucléique d'intérêt dans chaque zone réactionnelle d'un réseau de zones réactionnelles ;
b) détermination de la distribution des signaux optiques dans chaque zone réactionnelle ;
c) identification d'une zone réactionnelle comme invalide, si les signaux optiques dans la zone réactionnelle déterminés dans l'étape b) sont distribués de manière inégale dans la zone réactionnelle ; et
d) de calcul de la quantité ou de la concentration de l'acide nucléique d'intérêt sur la base des résultats de dPCR des zones réactionnelles non identifiées comme invalides dans l'étape c).

3. Procédé selon la revendication 1 ou 2, la zone réactionnelle étant identifiée comme invalide, si la distribution des signaux optiques dans la zone réactionnelle est **caractérisée par** (i) un écart-type au-dessus d'un seuil, (ii) une forme inappropriée de distribution, (iii) un écart d'un signal unique à partir de la moyenne des signaux au-dessus d'un seuil et/ou (iv) une moyenne des signaux s'écartant significativement de celle prévue.

4. Procédé selon l'une quelconque des revendications 1 à 3, les signaux optiques étant déterminés en utilisant un marqueur optique, préférablement un marqueur de contrôle de remplissage optiquement détectable ou une sonde PCR optiquement détectable.

5. Procédé selon l'une quelconque des revendications 1 à 4, la distribution étant déterminée par imagerie raster de chaque zone réactionnelle, particulièrement chaque raster représentant un signal optique constitué d'un nombre constant de pixels, spécialement d'un pixel, d'un dispositif optique, particulièrement d'un appareil photographique et/ou la distribution étant **caractérisée par** la moyenne des signaux optiques, la médiane, l'écart-type, le pas de signal le plus grand et le plus faible et/ou la forme de distribution.

6. Procédé selon l'une quelconque des revendications 1 à 5, les signaux optiques étant déterminés par procédé de détection en champ clair ou en champ sombre non fluorescent ou par fluorescence.

7. Procédé selon l'une quelconque des revendications 4 à 6, l'artéfact optique étant dû à la poussière, une égratignure, une projection de liquide, un cheveu, une fibre, une empreinte digitale, un remplissage incorrect d'une zone réactionnelle et/ou un défaut dans la structure du réseau.

8. Procédé selon l'une quelconque des revendications 4 à 7, le marqueur optique étant un marqueur par fluorescence.

9. Procédé selon la revendication 8, le marqueur optique
- ayant des propriétés de fluorescence et/ou d'absorbance différentes de celles des une ou plusieurs sondes dPCR ; et/ou
- ayant un décalage de Stokes d'au moins 100 nm, préférablement d'au moins 150 nm ; et/ou
- étant ATTO 430 LS ou ATTO 490 LS, préférablement ATTO 490 LS ; et/ou
- ayant une longueur d'onde d'excitation ou une longueur d'onde d'émission identique à un marqueur par fluorescence de sonde cible utilisé dans la dPCR.

10. Procédé selon l'une quelconque des revendications 1 à 9, l'acide nucléique d'intérêt
- étant un acide nucléique sélectionné dans le groupe constitué de l'ADN, de l'ADNc, de l'ARN et d'un mélange de ceux-ci ; et/ou
- étant indicatif d'un micro-organisme, d'une cellule, d'un virus, d'une bactérie, d'un champignon, d'une espèce de mammifère, d'un état ou d'une maladie génétique.

11. Procédé selon l'une quelconque des revendications 2 à 10, l'échantillon ayant été obtenu à partir d'une culture de cellules ou d'une source suspectée d'être contaminée, particulièrement d'un liquide corporel, de sang, de plasma sanguin, de sérum sanguin, d'urine, de bile, de liquide cérébro-spinal, d'un écouvillon, d'un échantillon clinique, d'un échantillon d'organe et d'un prélèvement de tissu ou d'un sujet, particulièrement d'un être humain, d'un animal et d'une plante, spécialement d'un être humain.

12. Procédé selon l'une quelconque des revendications 1 à 11, la zone réactionnelle étant une chambre miniaturisée d'un microréseau ou d'un nanoréseau, une chambre d'un dispositif microfluidique, un micropuits ou un nanopuits, sur une puce, dans un capillaire, sur une surface de liaison d'acide nucléique ou sur une bille, spécialement dans un microréseau ou sur une puce.

13. Procédé selon l'une quelconque des revendications 1 à 12, le réseau des zones réactionnelles comprenant
- au moins 100 zones réactionnelles, particulièrement au moins 1 000 zones réactionnelles, spécialement au moins 5 000 zones réactionnelles ; et/ou
- au plus 1 000 000 de zones réactionnelles, particulièrement au plus 100 000 zones réactionnelles,
- spécialement au plus 50 000, préférablement au plus 10 000 zones réactionnelles.

14. Procédé selon l'une quelconque des revendications 1 à 13, la dPCR impliquant l'utilisation d'une ou plusieurs sondes dPCR fluorescentes afin de détecter un ou plusieurs acide(s) nucléique(s) d'intérêt, particulièrement en combinaison avec un désactivateur ou comme balise moléculaire ou comme sonde d'hydrolyse et/ou particulièrement la sonde dPCR fluorescente comprenant de la fluorescéine, de la rhodamine et/ou de la cyanine.
